# EUROPEAN PATENT SPECIFICATION

(11) **EP 1 044 192 B1**
(45) Date of publication and mention of the grant of the patent: **18.08.2004**
(21) Application number: 98958910.6
(22) Date of filing: 20.11.1998
(51) Int. Cl.: C07D 249/08

(54) **PROCESS FOR PREPARING TRIAZOLE COMPOUNDS**
VERFAHREN ZUR HERSTELLUNG VON TRIAZOL DERIVATEN
PROCEDE DE PREPARATION DE COMPOSES TRIAZOLE

(30) Priority: 05.12.1997 IT MI972698
(43) Date of publication of application: 18.10.2000
(73) Proprietor: ZAMBON GROUP S.p.A., 36100 Vicenza (IT)
(72) Inventor: VILLA, Marco, I-20125 Milano (IT); BELLI, Aldo, I-20040 Cornate D'Adda (IT); PONZINI, Francesco, I-20146 Milano (IT)
(74) Representative: Longoni, Alessandra
(86) International application number: PCT/EP1998/007482
(87) International publication number: WO 1999/029676

(56) References cited:
- EP-A- 0 033 928
- EP-A- 0 251 775
- EP-A- 0 272 679
- EP-A- 0 711 775
- P.J.KOCIENSKI: "Protecting Groups" 1994 , G.THIEME , STUTTGART XP002098601 see page 96 - page 117
- HOUBEN-WEYL: "Methoden der Orgnasichen Chemi,Band 14a/1, O7O und O/S-Acetale" 1991 , G.THIEME , STUTTGART XP002098474 see page 162 - page 167
- HOUBEN-WEYL: "Methoden der Organischen Chemie,Bd.3,Sauerstoffverbindungen " 1965 , G.THIEME , STUTTGART XP002098475 see page 213 - page 220
- PATENT ABSTRACTS OF JAPAN vol. 009, no. 138 (C-286), 13 June 1985 & JP 60 025990 A (SHIONOGI SEIYAKU KK), 8 February 1985
- P.J.KOCIENSKI: "Protecting Groups" 1994 , G.THIEME , STUTTGART XP002098602 see page 46 - page 52
- PAQUETTE,L.A.: "Encyclopedia of Reagents of Organic Synthesis " 1995 , J.WILEY & SONS , CHICHESTER XP002098476 see page 316-8 - page 320-2
- MARCH,J.: "Advance Organic Chemistry,3rd.Ed." 1985 , J.WILEY & SONS , CHICHESTER XP002098477 see page 304 - page 307

## Description

The present invention refers to a process for the preparation of azole compounds endowed with antimycotic activity.

The compounds of formula I 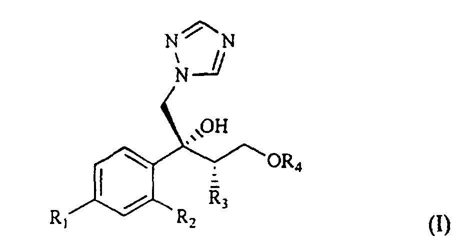 wherein
R₁ is chlorine, fluorine or trifluoromethyl;
R₂ is hydrogen, chlorine, fluorine or trifluoromethyl;
R₃ is C₁₋₄ alkyl; and
R₄ is a C₁₋₅ polyfluoroalkyl group containing at least two fluorine atoms and optionally other halogen atoms selected from chlorine and bromine;
and their salts with pharmaceutically acceptable acids, are known as antimycotic and antifungal agents.

The patent application WO 97/31903 (in the Applicant's name) shows a class of compounds which those of formula I above fall within, as broad spectrum antimycotics against human and animal pathogenic fungi. Such compounds are obtained from the intermediate of formula VIII 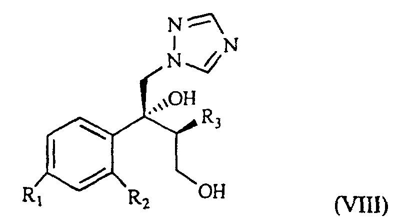 wherein R₁, R₂ and R₃ are as defined above, which reacted with the suitable polyfluoroalkyl derivative provides the desired compound of formula I.

It has been now found a new method for preparing the compounds of formula VIII constituting an alternative to the synthetic routes described in the above cited prior art.

Therefore the present invention refers to a method for preparing compounds of formula VIII as illustrated hereinbelow.

The synthesis of the compounds of formula VIII according to the present invention starts from the triol of formula II 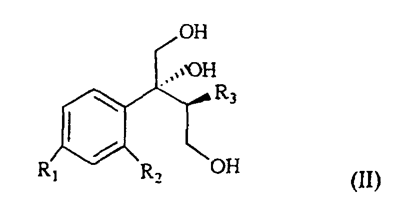 wherein R₁, R₂ and R₃ are as defined above. This is treated with a suitable ketone or aldehyde in acidic medium, for example in p-toluensulfonic acid, to selectively protect the two OH groups in α-position each other. For avoiding the formation of a further stereogenic centre it is preferred to use a symmetric ketone such as, for example, acetone or cyclohexanone. It is thus obtained the dioxolane of formula III 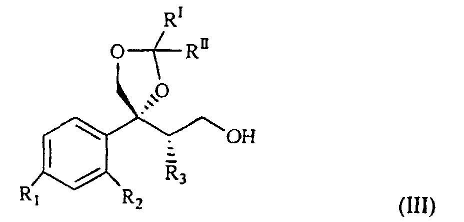 wherein R₁, R₂ and R₃ are as defined above, and R¹ and R¹¹ are depending on the aldehyde or ketone employed, which is duly protected on the free hydroxy moiety with an easily removable group, whereas resistant in acidic or weakly basic medium. Preferred examples of such groups are aryl-methyl such as benzyl or furfuryl-methyl optionally substituted which may be removed by hydrogenation. The protection reaction is effected, for example with an arylmethyl halide in the presence of an alkali metal hydride, for example sodium hydride, and provides the compound of formula IV 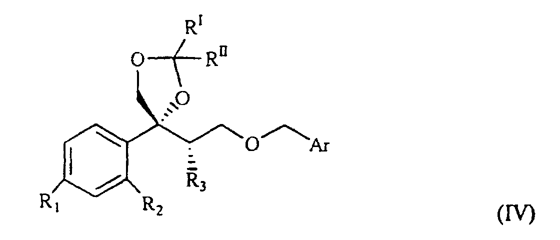 wherein R₁, R₂, R₃, R^{I} and R^{II} arc as defined above. and Ar is an optionally substituted aryl group. This is hydrolysed in strong acidic medium according to common methods, preferably in alcohol, for example ethanol, to give the compound of formula V 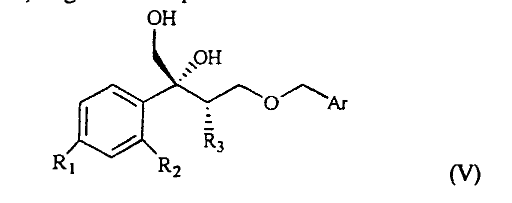 wherein R₁, R₂, R₃ and Ar are as defined above, which is functionalized on the primary hydroxy group by a leaving group Lg through reaction with a sulfonic acid chloride, such as methansulfonyl-chloride or tosyl chloride, or with a halogenating agent such as, for example, phosphorous tribromide, thionyl chloride or phosphorous pentachloride, thus obtaining the compound of formula VI 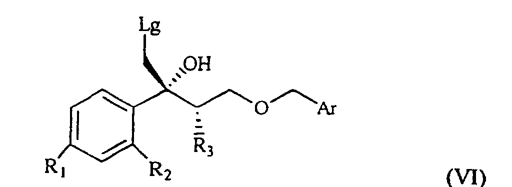 wherein R₁, R₂, R₃ and Ar are as defined above, Lg is halogen or a OSO₂R^{IV} group wherein R^{IV} is a (C₁₋₄)alkyl or an optionally methyl-substituted phenyl group. The compound of formula VI is treated with a salt of 1,2,4-triazole prepared previously or *in situ*, for example, with 1,2,4-triazole in the presence of sodium hydride and gives the compound of formula VII 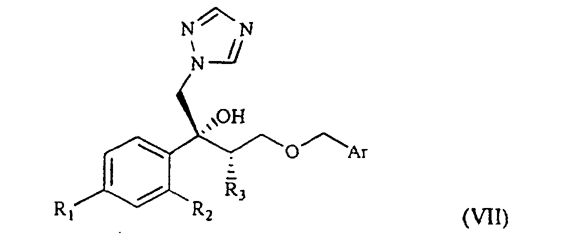 wherein R₁, R₂, R₃ and Ar are as defined above. Then the cleaving of the protected hydroxy group is effected by common techniques (see, for example, T.W. Greene and P.G.M. Wuts, Protective Groups in Organic Synthesis, John Wiley & Sons, New York), thus yielding the desired compound of formula VIII.

The intermediates V and VI are known (patent application EP 667346 in the name of Eisai). In particular, intermediate V is said to be obtained from one of the following compounds 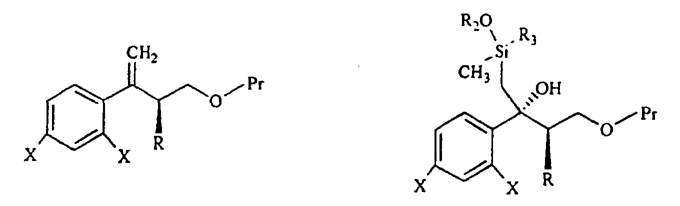 wherein Xs are halogen atoms, Pr is a protecting group, R is a lower alkyl group, R₃ is methyl or a lower alkoxy group and R₂ is a lower alkyl group. The intermediate of formula VI is obtained from the intermediate of formula V.

The substrate of formula II is known too and may be obtained both via literature methods and using a synthetic route claimed in a co-pending patent application filed in the same date of the present one by the Applicant (WO 99/29675), starting from a new compound claimed in another co-pending patent application filed in the same date of the present one by the Applicant.

Hereinbelow fulfilment examples of the present invention are provided.

### Example 1

### Synthesis of (2S,4R)-2,2-dimethyl-4-(2,4-dichlorophenyl)-4-(2-methyl-3-hydroxypropyl)-1,3-dioxolane

A solution of (2R,3S)-2-(2,4-dichlorophenyl)-3-methyl-1,2,4-trihydroxy-butane (2.65 g; 10 mmoles; enantiomeric excess: 97.8%) and p-toluensolfonic acid monohydrate (0.19 g; 1 mmole) in acetone (25 ml) was refluxed for 60 minutes, then cooled to 20°C, poured into water (100 ml) and extracted with toluene (25 ml x 3). The joined organic phases were washed with water (50 ml), anhydrified on dry Na₂SO₄. dried. 3.05 g Of (2S,4R)-2,2-dimethyl-4-(2,4-dichlorophenyl)-4-(2-methyl-3-hydroxypropyl)-1,3-dioxolane were obtained as a viscous oil.
¹H-NMR (300MHz, DMSO, δ=ppm, J=Hz): 0.55 (d, 3H, J=6.90): 1.10 (s, 3H); 1.40 (s, 3H); 2.23 (ddq, 1H, J=8.30, J=6.90, J=3.95); 3.17 (ddt, 1H, J=11.20, J=8.30, J=5.30); 3.77 (ddt, 1H, J=11.20, J=3.95, J=5.30); 4.06 (d, 1H, J=9.29); 4.50 (t, 1H, J=5.30); 4.72 (d, 1H, J=9.20); 7.43 (dd, 1H, J=8.50, J=2.16); 7.59 (d, 1H, J=2.16); 7.63 (d, 1H, J=8.50).

### Example 2

### Synthesis of (2S,4R)-2,2-dimethyl-4-(2,4-dichlorophenyl)-4-(2-methyl-3-phenylmethoxy)-propyl-1,3-dioxolane

A solution of crude (2S,4R)-2,2-dimethyl-4-(2,4-dichlorophenyl)-4-(2-methyl-3-hydroxypropyl)-1,3-dioxolane obtained in example 1 (3.05 g; 10 mmoles) in DMF (20 ml) cooled to 15°C under nitrogen was portionwise added with 60% NaH (0.5 g; 12.5 mmoles). The suspension was stirred for 30 minutes, then slowly added, in about 1 hour, with benzyl chloride (1.58 g; 12.5 mmoles) diluted in DMF (5 ml) and the mixture was kept under stirring at 15°C under nitrogen for further 20 hours, then poured into water (150 ml) and extracted with toluene (30 ml x 3). The organic phases were washed with water (50 ml), anhydrified over dry Na₂SO₄, dried. The crude was purified by flash chromatography (SiO₂; n-heptane/ethyl acetate 90/10) to give 3.82 g of (2S,4R)-2,2-dimethyl-4-(2,4-dichlorophenyl)-4-(2-methyl-3-phenylmethoxy)-propyl-1,3-dioxolane (yield 97%) as a colourless oil.
¹H-NMR (300 MHz, DMSO, δ=ppm, J=Hz): 0.59(d,3H, J=6.85); 1.05(s,3H); 1.41(s,3H); 2.55(ddq,1H, J=7.60, J=4.70, J=6.85); 3.26(dd,1H, J=9.50, J=7.60); 3.76(dd,1H, J=9.50, J=4.70); 4.05(d,1H, J=9.20); 4.46(d,1H, J=11.90); 4.52(d,1H, J=11.90); 4.75(d, 1H, J=9.20); 7.25-7.40(m, 5H); 7.44(dd, 1H, J=8.50, J=2.20); 7.62(d, 1H, J=2.20); 7.64(d, 1H, J=8.50).

### Example 3

### Synthesis of (2R,3S)-1-(2,4-dichlorophenyl)-1-[(2-phenylmethoxy-1-methyl)-ethyl]-ethandiol

A solution of (2S,4R)-2,2-dimethyl-4-(2,4-dichlorophenyl)-4-(2-methyl-3-phenylmethoxy)-propyl-1,3-dioxolane obtained in example 2 (3.75 g; 9.5 mmoles), 37% HCl (3.75 ml) and water (3.75 ml) in ethanol (37.5 ml) was refluxed for 24 hours, then cooled to room temperature, poured into water (150 ml) and extracted with toluene (30 ml x 3). The organic phases were washed with water (50 ml), anhydrified over dry Na₂SO₄, dried. The crude was purified by flash chromatography (SiO₂; n-heptane/ethyl acetate 75/25) to give 2.32 g of (2R,3S)-1-(2,4-dichlorophenyl)-1-[(2-phenylmethoxy-1-methyl)-ethyl]-ethandiol (yield 73%) as a colourless oil.
¹H-NMR (300MHz, DMSO, δ=ppm, J=Hz): 0.58 (d, 3H, J=7.00); 2.65 (ddq, 1H, J=6.90, J=5.20, J=7.00); 3.32 (dd, 1H, J=9.70, J=6.90); 3.76 (dd, 1H, J=9.70, J=5.20); 3.80 (dd, 1H, J=11.80, J=5.13); 4.25 (dd, 1H, J=11.80, J=5.50); 4.47 (t, 2H, J=14.10); 4.52 (dd, 1H, J=5.49, J=5.10); 5.00 (s, 1H); 7.25-7.5 (m, 7H); 7.76 (d, 1H, J=8.70).

### Example 4

### Synthesis of (2R,3S)-1-(2,4-dichlorophenyl)-[(2-phenylmethoxy-1-methyl)-ethyl]-ethandiol-2-p-toluensulfonate

A solution of (2R,3S)-1-(2,4-dichlorophenyl)-1-[(2-phenylmethoxy-1-methyl)-ethyl]-ethandiol obtained in example 3 (2.15 g; 6.05 mmoles) in pyridine (15 ml) cooled to 15-17°C under nitrogen was portionwise added in about 30 minutes with p-toluensulfonyl chloride (1.32 g; 6.9 mmoles) and the solution was stirred at 15°C under nitrogen for further 16 hours, then poured into water (100 ml), acidified to pH≤2 with 50% H₂SO₄ and extracted with toluene (30 ml x 3). The joined organic phases were washed with water (50 ml), anhydrified over dry Na₂SO₄, dried. The crude was purified by flash chromatography (SiO₂; n-heptane/ethyl acetate 85/15) to give 2.80 g of (2R,3S)-1-(2,4-dichlorophenyl)-[(2-phenylmethoxy-1-methyl)-ethyl]-ethandiol-2-p-toluensulfonate (yield 91%).
¹H-NMR (300MHz, DMSO, δ=ppm, J=Hz): 0.45(d, 3H, J=7.00); 2.40(s, 3H); 2.67(ddq, 1H, J=9.50, J=4.10, J=7.00); 3.26(dd, 1H, J=9.90, J=4.10); 3.72(dd, 1H, J=9.90, J=9.50); 4.42(s, 2H); 4.44(d, 1H, J=10.50); 4.85 (d, 1H, J=10.50); 5.85 (bs, 1H); 7.20-7.70 (m, 12H).

### Example 5

### Synthesis of (2R,3S)-2-(2,4-dichlorophenyl)-2-hydroxy-3-methyl-1-(1,2,4-triazol-1-yl)-4-benzyloxy-butane

A solution of 1,2,4-triazole (0.56 g, 8.1 mmoles) in DMF (6 ml) cooled to 16°C was portionwise added, under nitrogen, with 60% NaH (0.32 g; 8 mmoles) keeping the temperature at 15-20°C. The mixture was stirred at room temperature until the end of the gas generation, then heated to 125°C, slowly added in 30 minutes with a solution of (2R,3S)-1-(2,4-dichlorophenyl)-[(2-phenylmethoxy-1-methyl)-ethyl]-ethandiol-2-p-toluensulfonate obtained in example 4 (1.02 g; 2 mmoles) in DMF (2 ml), then kept under stirring at 125°C for further 90 minutes. The mixture was then poured into water (40 ml) and extracted with methylene chloride (20 ml x 3). The joined organic phases were washed with water (50 ml), anhydrified over dry Na₂SO₄, dried. The crude was purified by flash chromatography (SiO₂; hexane/ethyl acetate/methanol 80/20/5) to give 0.66 g of (2R,3S)-2-(2,4-dichlorophenyl)-2-hydroxy-3-methyl-1-(1,2,4-triazol-1-yl)-4-benzyloxy-butane (yield 82%).
¹H-NMR (300MHz, DMSO, δ=ppm, J=Hz): 0.69 (d, 3H, J=7.00); 3.07 (ddq, 1H, J=6.60, J=5.50, J=7.00); 3.45 (dd, 1H, J=9.50, J=5.50); 3.90 (dd, 1H, J=9.50, J=6.60); 4.53 (d, 1H, J=12.00); 4.60 (d, 1H, J=12.00); 4.74 (d, 1H, J=14.60); 5.18 (d, 1H, J=14.60); 5.65 (s, 1H); 7.18-7.50 (m, 9H); 7.60 (s, 1H); 8.22 (s, 1H).

### Example 6

### Synthesis of (2R,3S)-2-(2,4-dichlorophenyl)-3-methyl-1-(1H-1,2,4-triazol-1-yl)-2,4-butandiol

A solution of (2R,3S)-2-(2,4-dichlorophenyl)-2-hydroxy-3-methyl-1-(1,2,4-triazol-1-yl)-4-benzyloxy-butane obtained in example 5 (0.66 g; 1.64 mmoles) in 37% HCl (10 ml) was heated to 125°C for 30 minutes. After cooling to room temperature, the mixture was poured into ice (20 g), alkalinized to pH≥10 by 30% NaOH and extracted with methylene chloride (15 ml x 3). The joined organic phases were washed with water (50 ml), anhydrified over dry Na₂SO₄, dried. The crude was purified by flash chromatography (SiO_{2;} hexane/ethyl acetate/methanol 70/30/10) to give 0.44 g of (2R,3S)-2-(2,4-dichlorophenyl)-3-methyl-1-(1H-1,2,4-triazol-1-yl)-2,4-butandiol (yield 86%; enantiomeric excess: 97%).
¹H-NMR (300MHz, DMSO, δ=ppm, J=Hz): 0.56(d, 3H, J=7.00); 2.86(ddq, 1H, J=5.60, J=5.30, J=7.00); 3.58(ddd, 1H, J=11.00, J=5.30, J=5.00); 3.83(ddd, 1H, J=11.00, J=5.60, J=5.00); 4.78(d, 1H, J=4.60); 5.05(t, 1H, J=5.00); 5.2(d, 1H, J=4.60); 5.63 (s, 1H); 7.20 (dd, 1H, J=8.60, J=2.20); 7.36 (d, 1H, J=8.60); 7.47 (d, 1H, J=2.20); 7.70 (s, 1H); 8.25 s, 1H).

## Claims

1. Process for the preparation of a compound of formula VIII 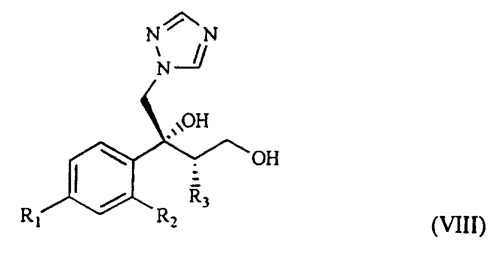 wherein
R₁ is chlorine, fluorine or trifluoromethyl;
R₂ is hydrogen, chlorine, fluorine or trifluoromethyl; and
R₃ is C₁₋₄ alkyl; which comprises the treatment of a triol of formula II 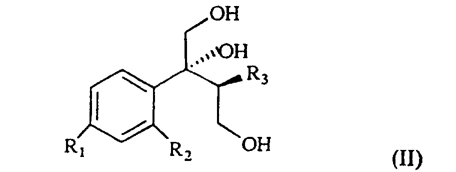
wherein R₁, R₂ and R₃ are as defined above, with a ketone or an aldehyde in strong acid to give the dioxolane of formula III 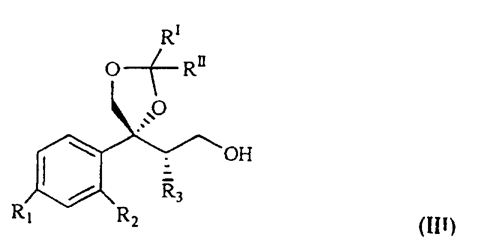 wherein R₁, R₂ and R₃ are as defined above, and R^{I} and R^{II} are depending on the aldehyde or ketone employed, which by treatment with an aryl-methyl halide optionally substituted on the aromatic ring and in the presence of an alkali metal hydride gives the compound of formula IV 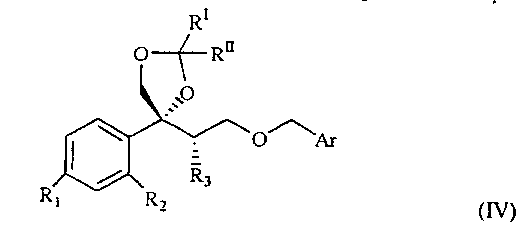 wherein R₁, R₂, R₃, R^{I} and R^{II} are as defined above, and Ar is optionally substituted aryl, which is hydrolyzed in acidic medium to give the compound of formula V 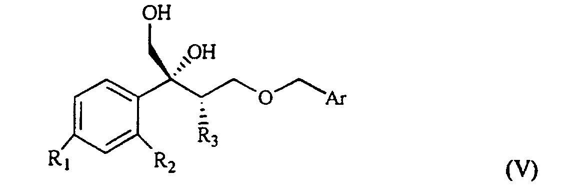 wherein R₁, R₂, R₃ and Ar are as defined above, which is functionalized on the primary hydroxy group by a leaving group Lg through a reaction with a sulfonic acid halide or a halogenating agent, thus yielding the compound of formula VI 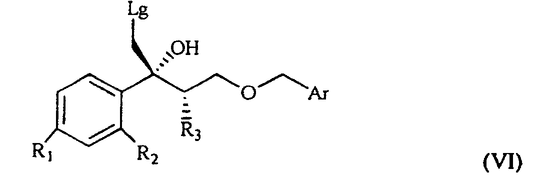 wherein R₁, R₂, R₃ and Ar are as defined above, and Lg is halogen or a OSO₂R^{IV} group wherein R^{IV} is a (C₁₋₄)alkyl or an optionally methyl-substituted phenyl group, which treated with a salt of 1,2,4-triazole prepared previously or *in situ*, yields the compound of formula VII 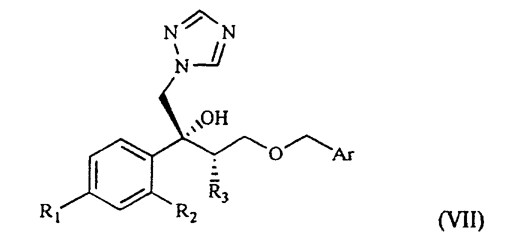 wherein R₁, R₂, R₃ and Ar are as defined above; then the cleaving of the protected hydroxy group is effected.

2. Process for the preparation of a compound of formula 1 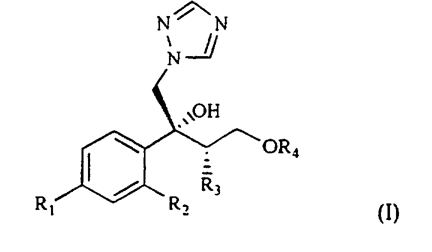 wherein
R₁ is chlorine, fluorine or trifluoromethyl;
R₂ is hydrogen, chlorine, fluorine or trifluoromethyl;
R₃ is C₁₋₄ alkyl; and
R₄ is a C₁₋₅ polyfluoroalkyl group containing at least two fluorine atoms and optionally other halogen atoms selected from chlorine and bromine;
and their salts with pharmaceutically acceptable acids, which comprises the treatment of a triol of formula II 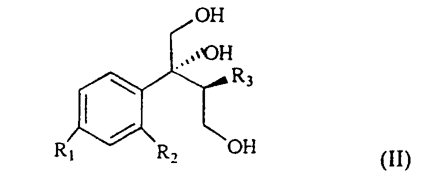 wherein R₁, R₂ and R₃ are as defined above, with a ketone or an aldehyde in strong acid to give the dioxolane of formula III 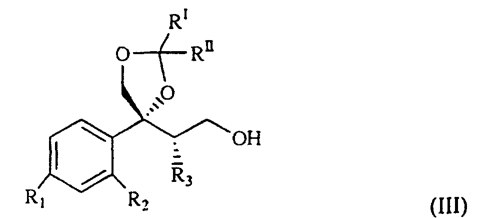 wherein R₁, R₂ and R₃ are as defined above, and R^{I} and R^{II} are depending on the aldehyde or ketone used, which by treatment with an aryl-methyl halide optionally substituted on the aromatic ring and in the presence of an alkali metal hydride gives the compound of formula IV 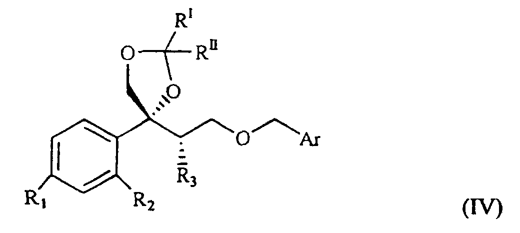 wherein R₁, R₂, R₃, R^{I} and R^{II} are as defined above, and Ar is optionally substituted aryl, which is hydrolyzed in acidic medium to give the compound of formula V 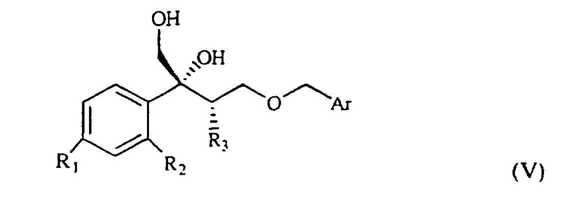 wherein R₁, R₂, R₃ and Ar are as defined above, which is functionalized on the primary hydroxy group by a leaving group Lg through a reaction with a sulfonic acid halide or a halogenating agent, thus yielding the compound of formula VI 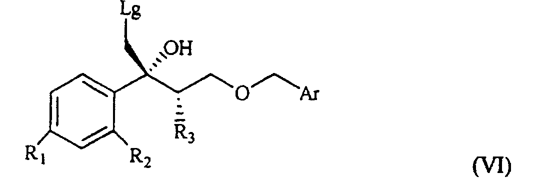 wherein R₁, R₂, R₃ and Ar are as defined above, and Lg is halogen or a OSO₂R^{IV} group wherein R^{IV} is a (C₁₋₄)alkyl or an optionally methyl-substituted phenyl group, which treated with a salt of 1,2,4-triazole prepared previously or *in situ*, yields the compound of formula VII 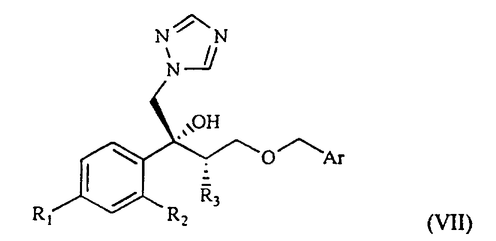 wherein R₁, R₂, R₃ and Ar are as defined above; then the cleaving of the protected hydroxy group is effected thus giving a compound of formula VIII 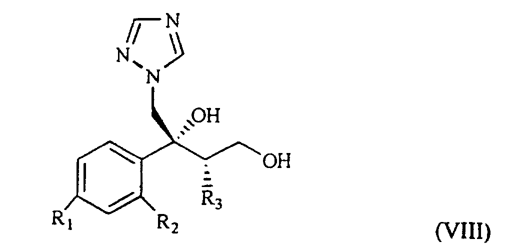 wherein R₁, R₂ and R₃ are as defined above, which is reacted with the suitable polyfluoroalkyl derivative.

3. Process for the preparation of a compound of formula VIII according to claim 1, which comprises the treatment of a triol of formula II 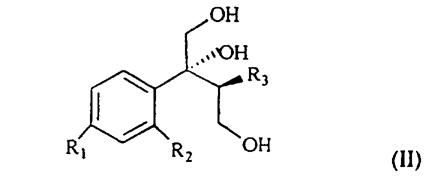 wherein R₁, R₂ and R₃ are as defined in claim 1, with a ketone or an aldehyde in strong acid to give the dioxolane of formula III 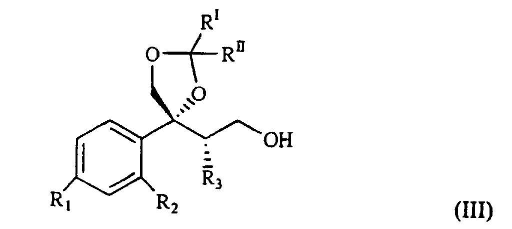 wherein R₁, R₂ and R₃ are as defined in claim 1, and R^{I} and R^{II} are depending on the aldehyde or ketone used, which by treatment with an aryl-methyl halide optionally substituted on the aromatic ring and in the presence of an alkali metal hydride gives the compound of formula IV 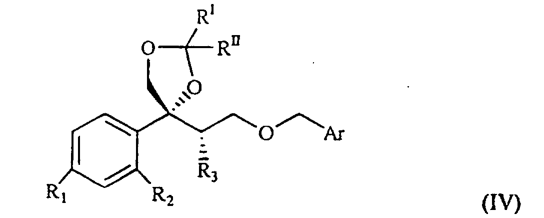 wherein R₁, R₂, R₃, R^{I} and R^{II} are as defined in claim 1, and Ar is optionally substituted aryl, which is hydrolyzed in acidic medium to give the compound of formula V 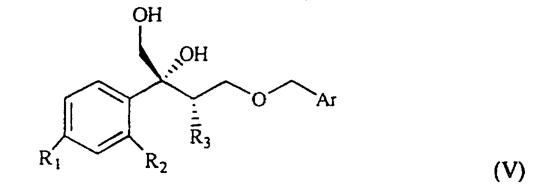 wherein R₁, R₂, R₃ and Ar are as defined in claim 1.

4. Process for the preparation of a compound of formula VIII according to claim 1, which comprises the treatment of a triol of formula II 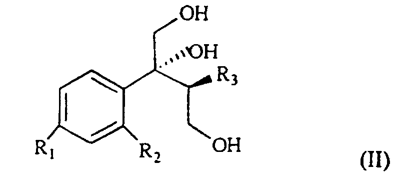 wherein R₁, R₂ and R₃ are as defined in claim 1, with a ketone or an aldehyde in strong acid to give the dioxolane of formula III 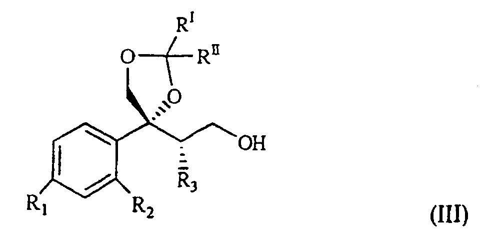 wherein R₁, R₂ and R₃ are as defined in claim 1, and R¹ and R¹¹ are depending on the aldehyde or ketone used, which by treatment with an aryl-methyl halide optionally substituted on the aromatic ring and in the presence of an alkali metal hydride gives the compound of formula IV 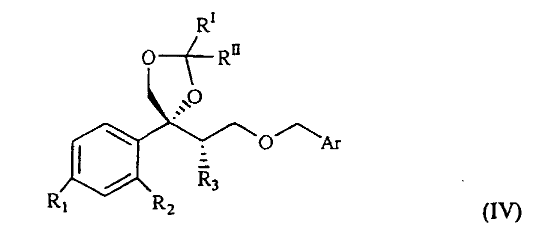 wherein R₁, R₂, R₃, R^{I}, R^{II} and Ar are as defined in claim 1, which is hydrolyzed in acidic medium to give the compound of formula V 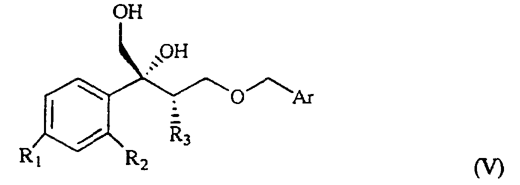 wherein R₁, R₂, R₃ and Ar are as defined in claim 1, which is functionalized on the primary hydroxy group by a leaving group Lg through a reaction with a sulfonic acid halide or a halogenating agent, thus yielding the compound of formula VI 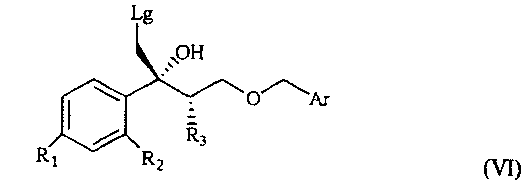 wherein R₁, R₂, R₃, Lg and Ar are as defined in claim 1.

5. A process for the preparation of a compound of formula VIII according to claim 1, which comprises the treatment of the compound of formula VI 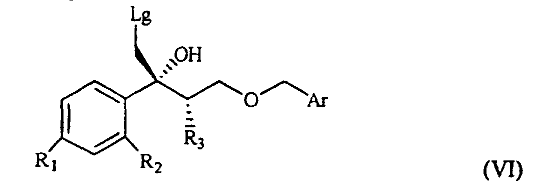 wherein R₁, R₂, R₃, Ar and Lg are as defined in claim 1, with a salt of 1,2,4-triazole prepared previously or *in situ*, to yield the compound of formula VII 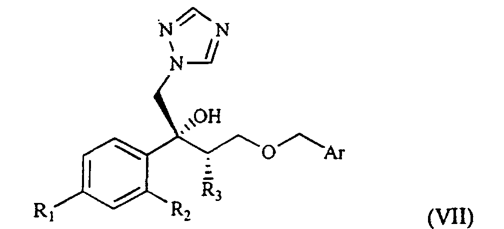 wherein R₁, R₂, R₃ and Ar are as defined in claim 1; then the cleaving of the protected hydroxy group.

## Patentansprüche

1. Verfahren zur Herstellung einer Verbindung der Formel VIII 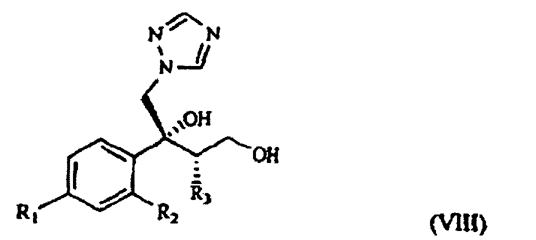 wobei
R₁ Chlor, Fluor oder Trifluormethyl ist;
R₂ Wasserstoff, Chlor, Fluor oder Trifluormethyl ist; und
R₃ C₁₋₄-Alkyl ist; umfassend die Behandlung eines Triols der Formel II 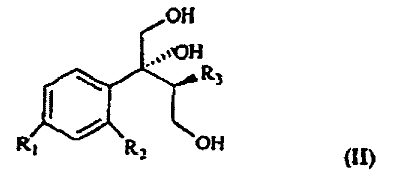
wobei R₁, R₂ und R₃ wie vorstehend definiert sind, mit einem Keton oder einem Aldehyd in starker Säure, um das Dioxolan der Formel III 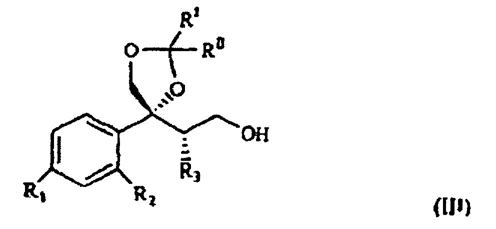 wobei R₁, R₂ und R₃ wie vorstehend definiert sind und R^{I} und R^{II} von dem eingesetzten Aldehyd oder Keton abhängig sind, bereitzustellen, das durch Behandlung mit einem gegebenenfalls am aromatischen Ring substituierten Arylmethylhalogenid und in Gegenwart eines Alkalimetallhydrids die Verbindung der Formel IV 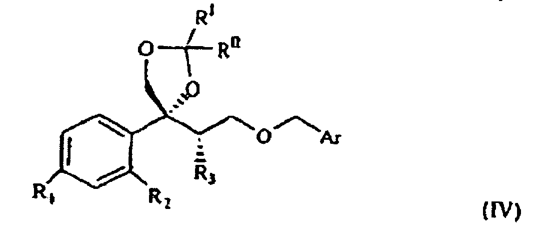 wobei R₁, R₂, R₃, R^{I} und R^{II} wie vorstehend definiert sind und Ar gegebenenfalls substituiertes Aryl ist, bereitstellt, die in saurem Medium hydrolysiert wird, um die Verbindung der Formel V 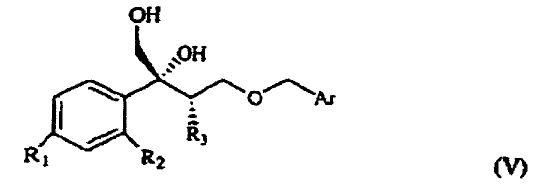 wobei R₁, R₂, R₃ und Ar wie vorstehend definiert sind, bereitzustellen, die an der primären Hydroxygruppe durch eine austretende Gruppe Lg durch Umsetzung mit einem Sulfonsäurehalogenid oder Halogenierungsmittel funktionalisiert wird, wodurch die Verbindung der Formel VI 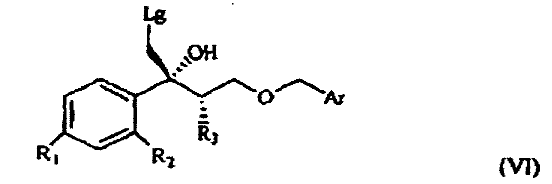 wobei R₁, R₂, R₃ und Ar wie vorstehend definiert sind und Lg Halogen oder eine OSO₂R^{IV}-Gruppe ist, wobei R^{IV} ein (C₁₋₄)-Alkyl oder eine gegebenenfalls mit Methyl substituierte Phenylgruppe ist, erhalten wird, die durch Behandlung mit einem vorher oder *in situ* hergestellten Salz von 1,2,4-Triazol die Verbindung der Formel VII 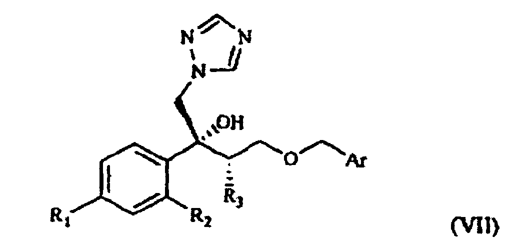 wobei R₁, R₂, R₃ und Ar wie vorstehend definiert sind, ergibt; dann die Abspaltung der geschützten Hydroxygruppe durchgeführt wird.

2. Verfahren zur Herstellung einer Verbindung der Formel I 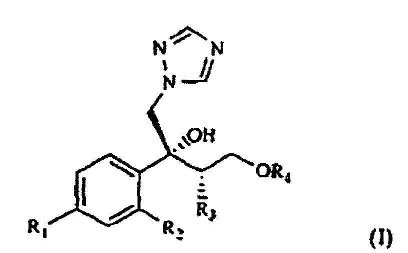 wobei
R₁ Chlor, Fluor oder Trifluormethyl ist;
R₂ Wasserstoff, Chlor, Fluor oder Trifluormethyl ist;
R₃ C₁₋₄-Alkyl ist; und
R₄ eine C₁₋₅-Polyfluoralkylfgruppe ist, die mindestens zwei Fluoratome und gegebenenfalls andere Halogenatome, ausgewählt aus Chlor und Brom, enthält;
und deren Salze mit pharmazeutisch verträglichen Säuren, umfassend die Behandlung eines Triols der Formel II 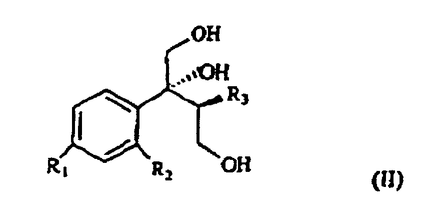 wobei R₁, R₂ und R₃ wie vorstehend definiert sind, mit einem Keton oder einem Aldehyd in starker Säure, um ein Dioxolan der Formel III 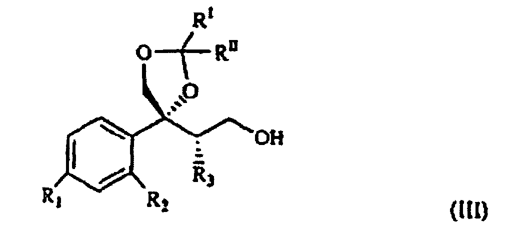 wobei R₁, R₂ und R₃ wie vorstehend definiert sind und R^{I} und R^{II} von dem verwendeten Aldehyd oder Keton abhängig sind, bereitzustellen, das durch Behandlung mit einem gegebenenfalls am aromatischen Ring substituierten Arylmethylhalogenid und in Gegenwart eines Alkalimetallhydrids die Verbindung der Formel IV 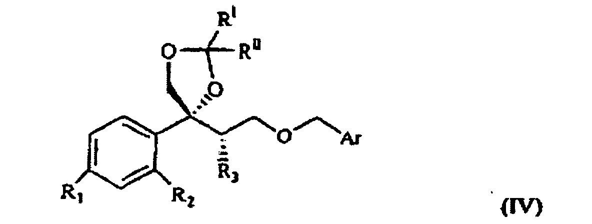 wobei R₁, R₂, R₃ R^{I} und R^{II} wie vorstehend definiert sind und Ar gegebenenfalls substituiertes Aryl ist, bereitstellt, die in saurem Medium hydrolysiert wird, um die Verbindung der Formel V 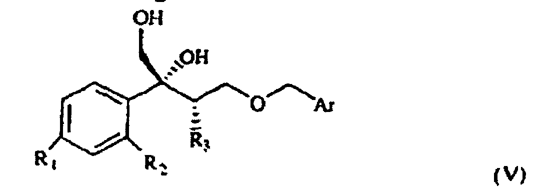 wobei R₁, R₂, R₃ und Ar wie vorstehend definiert sind, bereitzustellen, die an der primären Hydroxygruppe durch eine austretende Gruppe Lg durch Umsetzung mit einem Sulfonsäurehalogenid oder einem Halogenierungsmittel funktionalisiert wird, womit die Verbindung der Formel VI 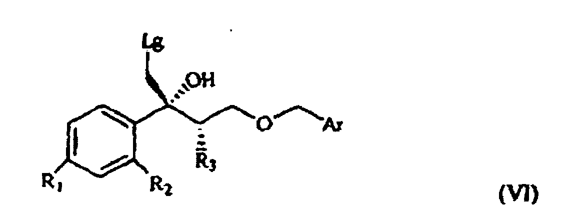 wobei R₁, R₂, R₃ und Ar wie vorstehend definiert sind und Lg Halogen oder eine OSO₂R^{IV}-Gruppe ist, wobei R^{IV} ein (C₁₋₄)-Alkyl oder eine gegebenenfalls mit Methyl substituierte Phenylgruppe ist, erhalten wird, die durch Behandlung mit einem vorher oder *in situ* hergestellten Salz von 1,2,4-Triazol die Verbindung der Formel VII 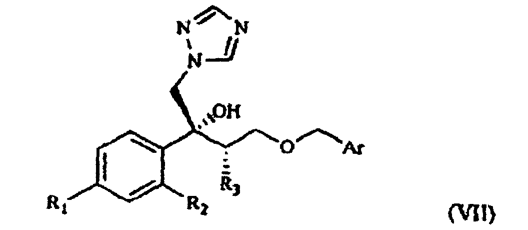 wobei R₁, R₂, R₃ und Ar wie vorstehend definiert sind, ergibt; dann die Abspaltung der geschützten Hydroxygruppe durchgeführt wird, wodurch eine Verbindung der Formel VIII 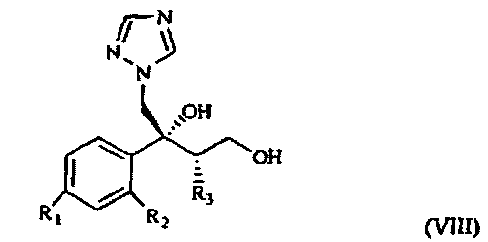 wobei R₁, R₂ und R₃ wie vorstehend definiert sind, bereitgestellt wird, die mit dem geeigneten Polyfluorderivat umgesetzt wird.

3. Verfahren zur Herstellung einer Verbindung der Formel VIII nach Anspruch 1, umfassend die Behandlung eines Triols der Formel II 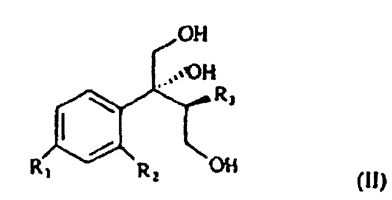 wobei R₁, R₂ und R₃ wie in Anspruch 1 definiert sind, mit einem Keton oder einem Aldehyd in starker Säure, um ein Dioxolan der Formel III 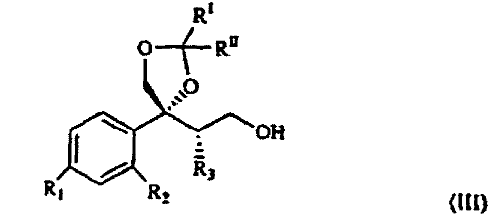 wobei R₁, R₂ und R₃ wie in Anspruch 1 definiert sind und R^{I} und R^{II} von dem verwendeten Aldehyd oder Keton abhängig sind, bereitzustellen, das durch Behandlung mit einem gegebenenfalls am aromatischen Ring substituierten Arylmethylhalogenid und in Gegenwart eines Alkalimetallhydrids die Verbindung der Formel IV 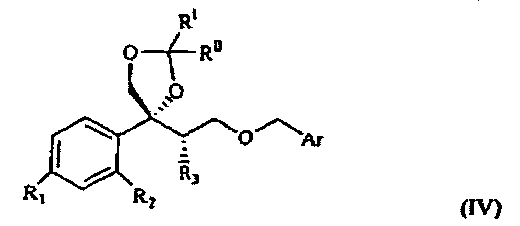 wobei R₁, R₂, R₃ R^{I} und R^{II} wie in Anspruch 1 definiert sind und Ar gegebenenfalls substituiertes Aryl ist, bereitstellt, die in saurem Medium hydrolysiert wird, um die Verbindung der Formel V 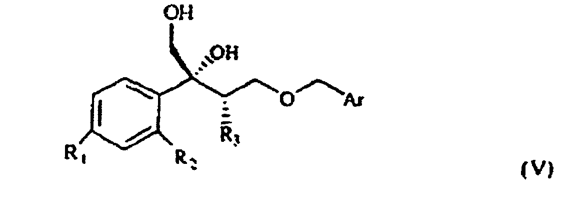 wobei R₁, R₂, R₃ und Ar wie in Anspruch definiert sind, bereitzustellen.

4. Verfahren zur Herstellung einer Verbindung der Formel VIII nach Anspruch 1, umfassend die Behandlung eines Triols der Formel II 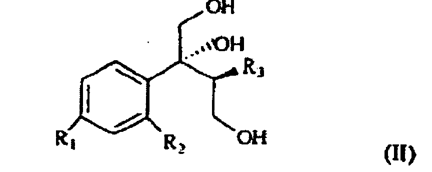 wobei R₁, R₂ und R₃ wie in Anspruch 1 definiert sind, mit einem Keton oder einem Aldehyd in starker Säure, um ein Dioxolan der Formel III 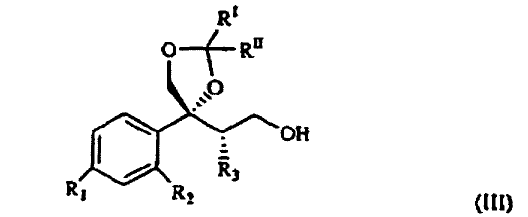 wobei R₁, R₂ und R₃ wie in Anspruch 1 definiert sind und R^{I} und R^{II} von dem verwendeten Aldehyd oder Keton abhängig sind, bereitzustellen, das durch Behandlung mit einem gegebenenfalls am aromatischen Ring substituierten Arylmethylhalogenid und in Gegenwart eines Alkalimetallhydrids die Verbindung der Formel IV 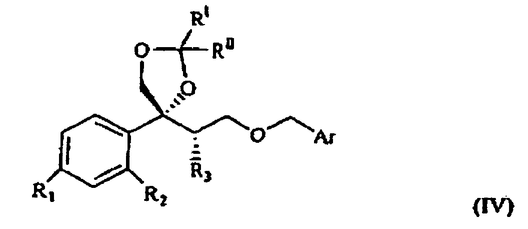 wobei R₁, R₂, R₃ R^{I}, R^{II} und Ar wie in Anspruch 1 definiert sind, bereitstellt, die in saurem Medium hydrolysiert wird, um die Verbindung der Formel V 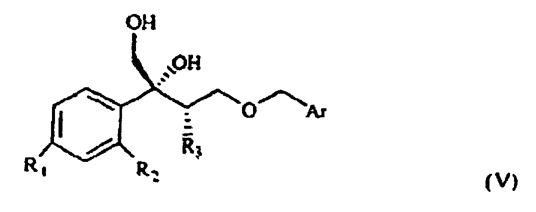 wobei R₁, R₂, R₃ und Ar wie in Anspruch definiert sind, bereitzustellen, die an der primären Hydroxygruppe durch eine austretende Gruppe Lg durch Umsetzung mit einem Sulfonsäurehalogenid oder einem Halogenierungsmittel funktionalisiert wird, wodurch die Verbindung der Formel VI 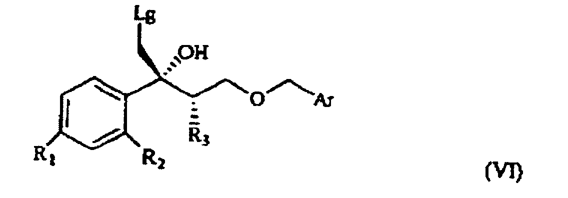 wobei R₁, R₂, R₃, Lg und Ar wie in Anspruch 1 definiert sind, erhalten wird.

5. Verfahren zur Herstellung einer Verbindung der Formel VIII nach Anspruch 1, umfassend die Behandlung der Verbindung der Formel VI 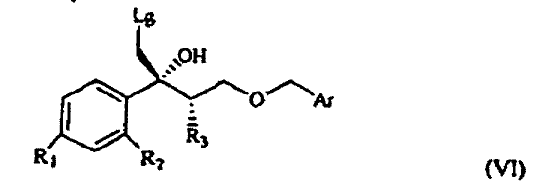 wobei R₁, R₂, R₃, Ar und Lg wie in Anspruch 1 definiert sind, mit einem vorher oder *in situ* hergestellten Salz von 1,2,4-triazol, um die Verbindung der Formel VII 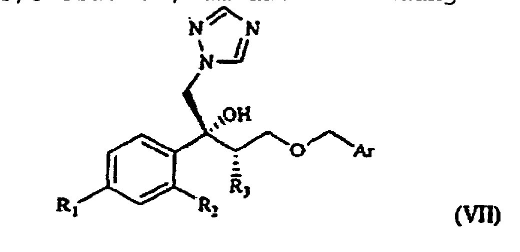 wobei R₁, R₂, R₃ und Ar wie in Anspruch 1 definiert sind, zu erhalten; dann das Abspalten der geschützten Hydroxygruppe.

## Revendications

1. Procédé pour la préparation d'un composé de formule VII: 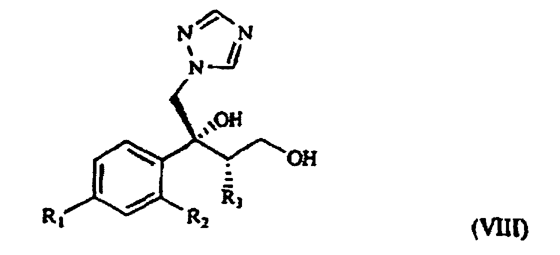 selon laquelle R₁ est du chlore, du fluor, ou du trifluorométhyle ;
R₂ est de l'hydrogène, du chlore ou du trifluorométhyle; et R₃ est un alkyle C₁₋₄ ; qui comprend le traitement d'un triol de formule II : 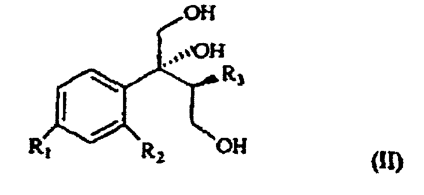 selon laquelle R₁, R₂, et R₃ sont tels que définis précédemment, avec une cétone ou un aldéhyde comme acide fort pour fournir le dioxolane de formule III : 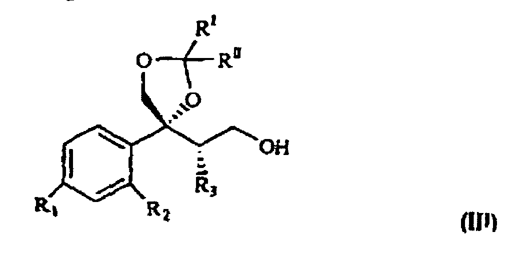 selon laquelle R₁, R₂, et R₃ sont tels que définis précédemment, et R' et R" dépendent de l'aldéhyde ou de la cétone utilisé, qui par traitement avec un halogénure aryle-méthyle éventuellement substitué sur l'anneau aromatique et en présence d'un métal hydride alcalin fournit le composé de formule IV : 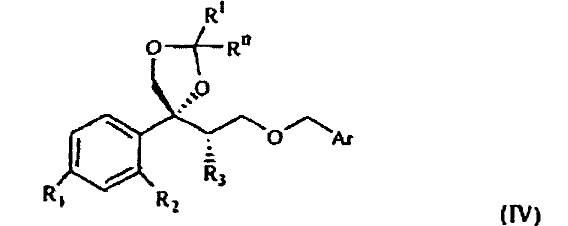 selon laquelle R₁, R₂, R₃, R' et R" sont tels que définis précédemment, et Ar est éventuellement de l'aryle substitué, qui est hydrolysé en milieu acide pour fournir le composé de formule V : 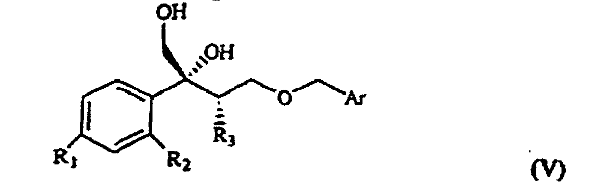 selon laquelle R₁, R₂ et R₃ sont tels que définis précédemment, qui est fonctionnalisé à partir du groupe hydroxy primaire par un groupe sortant Lg moyennant une réaction avec un halogénure d'acide sulfonique ou un agent halogène, fournissant ainsi le composé de formule VI : 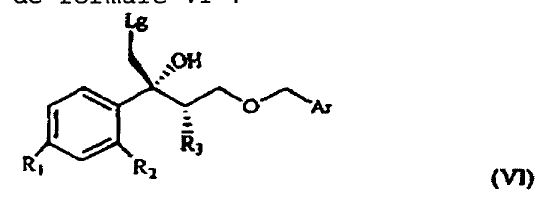 selon laquelle R₁, R₂, R₃ et Ar sont tels que définis précédemment, et Lg est un halogène ou un groupe OSO₂R^{iv} selon lequel R^{iv} est un alkyle C₁₋₄ ou un groupe phényle éventuellement substitué par du méthyle qui, lorsque traité avec un sel de 1,2,4-triazole préparé antérieurement ou in situ, fournit un composé de formule VII : 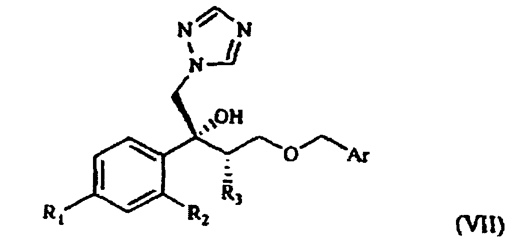 selon laquelle R₁, R₂, R₃ et Ar sont tels que définis précédemment; suivi du clivage du groupe hydroxy protégé.

2. Procédé de préparation d'un composé de formule I : 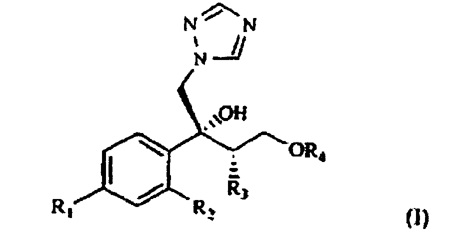 selon laquelle R₁ est du chlore, du fluor ou du trifluorométhyle ;
R₂ est de l'hydrogène, du chlore, du fluor ou du trifluorométhyle;
R₃ est un alkyle C₁₋₄; et
R₄ est un groupe polyfluoroalkyle C₁₋₅ contenant au moins deux atomes de fluor, et éventuellement des atomes d'halogène choisis parmi le chlore et le brome;
et leurs sels avec acides acceptables au niveau pharmaceutique, qui comprend le traitement d'un triol de formule II : 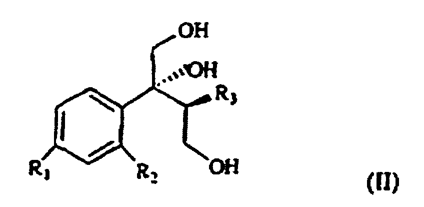 selon laquelle R₁, R₂ et R₃ sont tels que définis précédemment, avec une cétone ou un aldéhyde comme acide fort pour fournir le dioxolane de formule III : 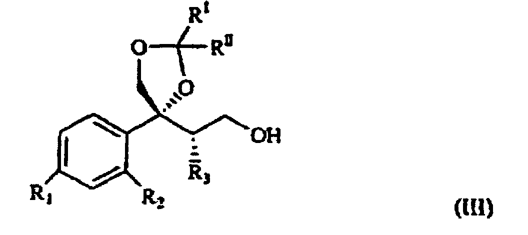 selon laquelle R₁, R₂ et R₃ sont tels que définis précédemment, et R' et R" dépendent de la cétone ou de l'aldéhyde utilisé, qui par traitement avec un halogénure aryle-méthyle éventuellement substitué sur l'anneau aromatique et en présence d'un métal hydride alcalin fournit le composé de formule IV : 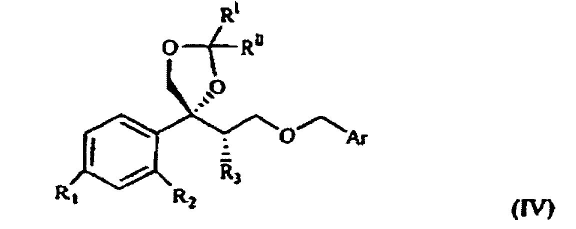 selon laquelle R₁, R₂, R₃, R' et R" sont tels que définis précédemment, et Ar est de l'aryle éventuellement substitué, qui est hydrolysé en milieu acide pour fournir le composé de formule V : 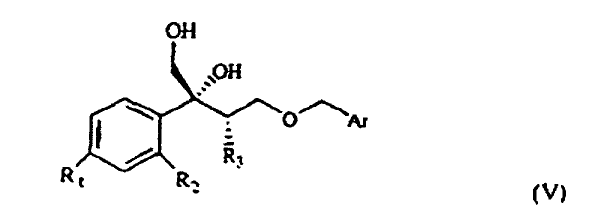 selon laquelle R₁, R₂, R₃ et Ar sont tels que définis précédemment, qui est fonctionnalisé à partir du groupe hydroxy primaire par un groupe sortant Lg moyennant une réaction avec un halogénure d'acide sulfonique ou un agent halogène, fournissant ainsi le composé de formule VI : 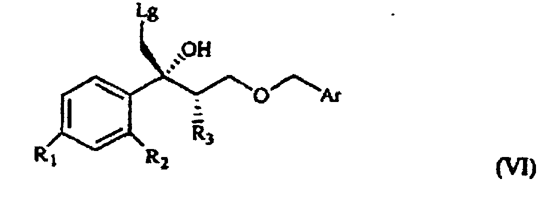 selon laquelle selon laquelle R₁, R₂, R₃ et Ar sont tels que définis précédemment, et Lg est un halogène ou un groupe OSO₂R^{iv} selon lequel R^{iv} est un alkyle C₁₋₄ ou un groupe phényle éventuellement substitué par du méthyle, qui, lorsque traité avec un sel de 1,2,4-triazole préparé antérieurement ou *in situ*, fournit un composé de formule VII : 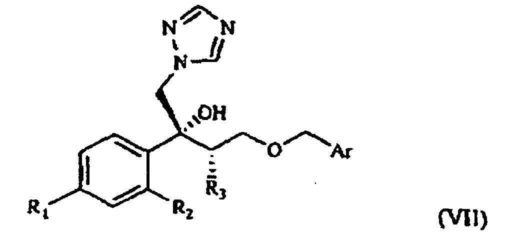 selon laquelle R₁, R₂, R₃ et Ar sont tels que définis précédemment; suivi du clivage du groupe hydroxy protégé qui fournit ainsi le composé de formule VIII : 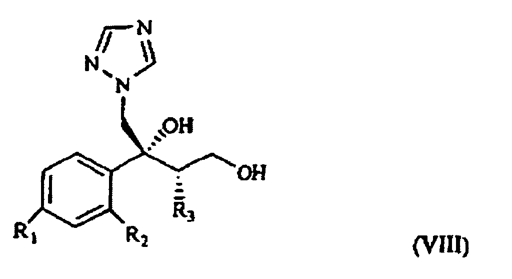 selon laquelle R₁, R₂ et R₃ sont tels que définis précédemment, qui est traité avec un dérivé polyfluoroalkyle convenable.

3. Procédé pour la préparation d'un composé de formule VIII selon la revendication 1, qui comprend le traitement d'un triol de formule II : 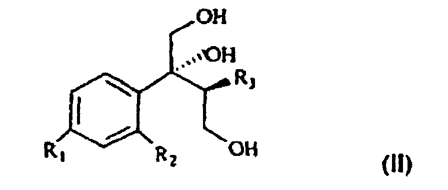 selon laquelle R₁, R₂ et R₃ sont tels que définis à la revendication 1, avec une cétone ou un aldéhyde comme acide fort pour fournir du dioxolane de formule III: 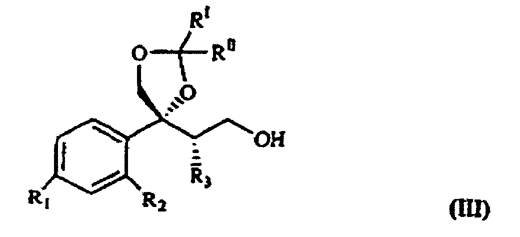 selon laquelle R₁, R₂ et R₃ sont tels que définis à la revendication 1, et R' et R" dépendent de la cétone ou de l'aldéhyde utilisé, qui par traitement avec un halogénure aryle-méthyle éventuellement substitué sur l'anneau aromatique et en présence d'un métal hydride alcalin fournit le composé de formule IV : 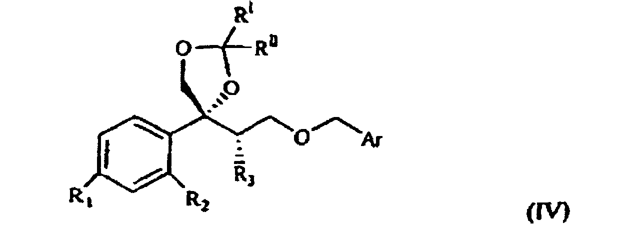 selon laquelle R₁, R₂, R₃, R' et R" sont tels que définis à la revendication 1, et Ar est de l'aryle éventuellement substitué, qui est hydrolysé en milieu acide pour fournir le composé de formule V : 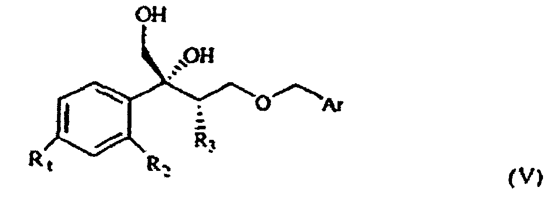 selon laquelle R₁, R₂, R₃, et Ar sont tels que définis à la revendication 1.

4. Procédé de préparation d'un composé de formule VIII selon la revendication 1, qui comprend le traitement d'un triol de formule II : 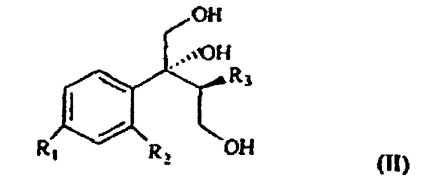 selon laquelle R₁, R₂ et R₃ sont tels que définis à la revendication 1, avec une cétone ou un aldéhyde comme acide fort pour fournir du dioxolane de formule III: 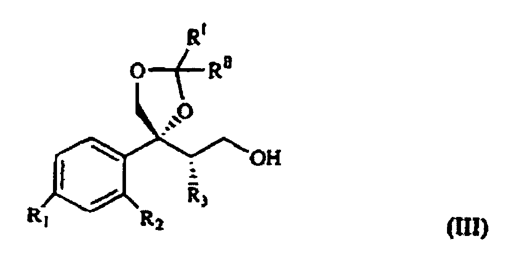 selon laquelle R₁, R₂ et R₃ sont tels que définis à la revendication 1, et R' et R" dépendent de la cétone ou de l'aldéhyde utilisé, qui par traitement avec un halogénure aryle-méthyle éventuellement substitué sur l'anneau aromatique et en présence d'un métal hydride alcalin fournit le composé de formule IV : 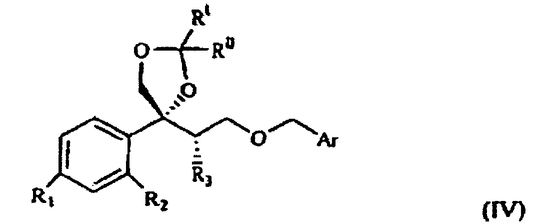 selon laquelle R₁, R₂, R₃, R', R" et Ar sont tels que définis à la revendication 1, qui est hydrolysé en milieu acide pour fournir le composé de formule V : 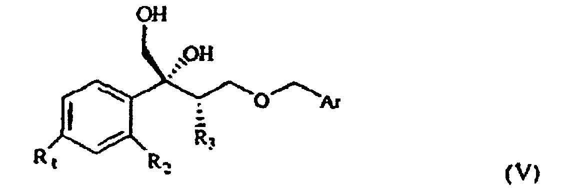 selon laquelle R₁, R₂, R₃, et Ar sont tels que définis à la revendication 1, qui est fonctionnalisé à partir du groupe hydroxy primaire par un groupe sortant Lg moyennant une réaction avec un halogénure d'acide sulfonique ou un agent halogène, fournissant ainsi le composé de formule VI : 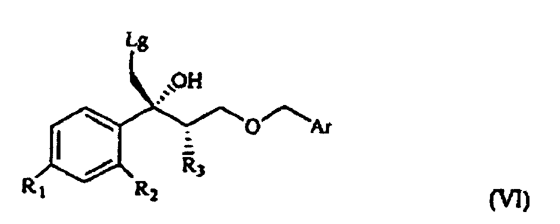 selon laquelle R₁, R₂, R₃ et Ar sont tels que définis à la revendication 1.

5. Procédé pour la préparation d'un composé de formule VIII selon la revendication 1, qui comprend le traitement du composé de formule VI : 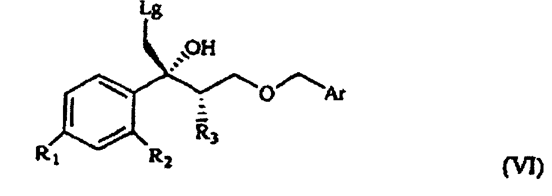 selon laquelle R₁, R₂, R₃, Ar et Lg sont tels que définis à la revendication 1, avec un sel de 1,2,4-triazole préparé antérieurement ou *in situ* pour fournir le composé de formule VII : 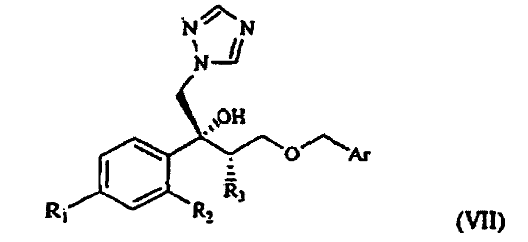 selon laquelle R₁, R₂, R₃ et Ar sont tels que définis à la revendication 1 ; suivi du clivage du groupe hydroxy protégé.
